# EUROPÄISCHE PATENTSCHRIFT

(11) **EP 2 668 500 B1**
(45) Veröffentlichungstag und Bekanntmachung des Hinweises auf die Patenterteilung: **13.04.2016**
(21) Anmeldenummer: 12710162.4
(22) Anmeldetag: 21.02.2012
(51) Int. Cl.: G01N 33/543

(54) **MINIATURISIERTE MAGNETISCHE DURCHFLUSSZYTOMETRIE**
MINIATURIZED MAGNETIC FLOW CYTOMETRY
CYTOMÉTRIE DE FLUX MAGNÉTIQUE MINIATURISÉE

(30) Priorität: 28.02.2011 DE 102011004805
(43) Veröffentlichungstag der Anmeldung: 04.12.2013
(73) Patentinhaber: Siemens Aktiengesellschaft, 80333 München (DE)
(72) Erfinder: HAYDEN, Oliver, 91074 Herzogenaurach (DE); HELOU, Michael Johannes, 93051 Regensburg (DE); REISBECK, Mathias, 93083 Obertraubling (DE); TEDDE, Sandro Francesco, 91058 Erlangen (DE)
(86) Internationale Anmeldenummer: PCT/EP2012/052901
(87) Internationale Veröffentlichungsnummer: WO 2012/116906

(56) Entgegenhaltungen:
- CARR C ET AL: "Magnetic Sensors for Bioassay: HTS SQUIDs or GMRs?", IEEE TRANSACTIONS ON APPLIED SUPERCONDUCTIVITY, IEEE SERVICE CENTER, LOS ALAMITOS, CA, US, Bd. 17, Nr. 2, 1. Juni 2007 (2007-06-01), Seiten 808-811, XP011188120, ISSN: 1051-8223, DOI: 10.1109/TASC.2007.897369
- INGLIS D W ET AL: "Continuous microfluidic immunomagnetic cell separation", APPLIED PHYSICS LETTERS, AIP, AMERICAN INSTITUTE OF PHYSICS, MELVILLE, NY, US, Bd. 85, Nr. 21, 22. November 2004 (2004-11-22), Seiten 5093-5095, XP002612323, ISSN: 0003-6951, DOI: 10.1063/1.1823015
- YANG SUNG-YI ET AL: "Micro flow cytometry utilizing a magnetic bead-based immunoassay for rapid virus detection", BIOSENSORS AND BIOELECTRONICS, ELSEVIER BV, NL, Bd. 24, Nr. 4, 2008, Seiten 855-862, XP002674649, ISSN: 0956-5663
- LIU CHENGXUN ET AL: "Cell manipulation with magnetic particles toward microfluidic cytometry", JOURNAL OF APPLIED PHYSICS, AMERICAN INSTITUTE OF PHYSICS. NEW YORK, US, Bd. 105, Nr. 10, 19. Mai 2009 (2009-05-19) , Seiten 102014-102014, XP012125274, ISSN: 0021-8979, DOI: 10.1063/1.3116091
- MUJIKA M ET AL: "Magnetoresistive immunosensor for the detection of Escherichia coli O157:H7 including a microfluidic network", BIOSENSORS AND BIOELECTRONICS, ELSEVIER BV, NL, Bd. 24, Nr. 5, 1. Januar 2009 (2009-01-01) , Seiten 1253-1258, XP025868513, ISSN: 0956-5663, DOI: 10.1016/J.BIOS.2008.07.024 [gefunden am 2008-07-25]

## Beschreibung

Die vorliegende Erfindung betrifft die magnetische Zelldetektion im Durchfluss.

Im Bereich der Zellmessung und Zelldetektion sind neben optischen Messverfahren, wie Streulicht- oder Fluoreszenzmessung, auch magnetische Detektionsverfahren bekannt, bei denen die zu detektierende Zellsorte mittels magnetischer Labels markiert wird.

Insbesondere sind zur magnetbasierten Messung Verfahren bekannt, bei denen magnetisch markierte Zellen mittels Magnetophorese aus einer komplexen Zellsuspension, z.B. einer Blutprobe, aussortiert werden. Dazu müsste diese komplexe Suspension erst dementsprechend aufbereitet werden, dass zu detektierenden Zellen daraus abgetrennt werden können. Die magnetische Markierung erfolgt insbesondere dadurch, dass zellspezifische Marker in die komplexe Zellprobe eingeführt werden. Magnetophorese wird bisher zur Sortierung von magnetisch markierten Zellen oder allgemein magnetischen Partikeln verwendet.

Im Bereich der magnetoresistiven Sensorik für Zelldetektion ist es aber auch möglich, magnetisch markierte Zellen in einer komplexen Suspension im Durchfluss dynamisch zu zählen. Dazu ist es wichtig, dass die Zellen vereinzelt nacheinander über den Sensor fließen und dass die magnetisch markierten Zellen in ausreichender Nähe zum magnetoresistiven Sensor über diesen hinweggeführt werden.

In einem magnetischen Durchflusszytometer werden markierte Zellen in einem Kanal daher oberflächennah über einen Magnetsensor transportiert. Die Nähe einer magnetisch markierten Zelle zum Sensor ist entscheidend, da das magnetische Streufeld der magnetischen Marker, anhand dessen die markierte Zelle letztlich durch den Sensor detektiert wird, mit der dritten Potenz zum Abstand abfällt.

Um sicherzustellen, dass eine markierte Zelle den Sensor in unmittelbarer Nähe passiert, ist es grundsätzlich denkbar, den Durchmesser des Kanals, durch den die Zellprobe strömt, möglichst klein zu gestalten. D.h. im Extremfall ist der Kanaldurchmesser gerade so groß, dass einzelne Zellen passieren können. Problematisch wirkt sich hierbei natürlich aus, dass die Anwesenheit von Verunreinigungen bzw. störenden Partikeln sehr schnell zu einer Verstopfung des Kanals führt.

Wird der Kanal dagegen größer ausgelegt, steigt auch die Wahrscheinlichkeit, dass einige markierte Zellen den Sensor außerhalb von dessen Reichweite passieren und somit nicht detektiert werden. Dem kann dadurch entgegen gewirkt werden, dass die magnetisch markierten Zellen am Sensor angereichert werden: Es hat sich gezeigt, dass sich eine möglichst lange Anreicherungsstrecke durch einen Mikrofluidikkanal von bis zu 1 cm Länge positiv darauf auswirkt, dass am Ende der Anreicherungsstrecke nahezu 100 % der magnetisch markierten Zellen aus der komplexen Suspension so am Kanalboden angereichert sind, dass eine Erfassung durch einen Magnetsensor möglich ist. Eine derart lange Anreicherungsstrecke auf einem Halbleitersubstrat, auf welchem das magnetoresistive Bauteil ausgebildet ist anzuordnen, führt jedoch zu einem hohen Aspektverhältnis des Substrats, welches neben hohen Kosten für die Gesamtfläche des Halbleitersubstrats, insbesondere für Siliziums Dies auch zu Problemen bei der Prozessierung im Herstellungsprozess führt. Je höher die Geschwindigkeit des Durchflusses und je höher die Zellkonzentration in der Probe, desto länger muss die Ausrichtungsstrecke gewählt werden, um zu einer ausreichend ausreichenden Anreicherung der magnetisch markierten Zellen zum Zeitpunkt des Sensorübertritts zu gewährleisten.

Der Artikel "Magnetic Sensors for Bioassay: HTS SQUIDs or GMRs?"; C. Carr et al.; IEEE Transactions on applied Superconductivity, Vol. 17, Nr. 2, S. 808-811 betrifft Sensoren zur Erfassung magnetischer Nanopartikel. Als magnetische Sensoren werden SQUID-Sensoren und GMR-Sensoren genannt.

In dem Artikel "Continious microfluidic immunomagnetic cell separation"; D. Inglis et al.; Applied physics letters, vol. 85, Nr. 21, S. 5093-5095 ist eine Trennung von magnetisch markierten Zellen aus einem Volumenstrom betrieben. Hierzu wird der Volumenstrom über ein Siliziumsubstrat mit integrierten magnetischen Streifen geleitet.

Der Bericht "micro flow cytometry utilizing a magnetic beadbased immunoassay for rapid virus detection"; Sung-Yi Yang et al.; Biosensors and Bioelectronics 24 (2008), S. 855-862 beschreibt ein Verfahren zur Detektion von Viren durch Anbindung der Viren an magnetische "beads". Die Viren werden anschließend mittels Mikroflusszytometrie detektiert.

In dem Artikel "Cell manipulation with magnetic particles towards microfluidic cytometry"; Chengyun Liu et al.; Journal of applied Physics, Bd. 105, Nr. 10 (2009), S. 102014-1 - 102014-11 ist ein Verfahren zur automatischen Sortierung und Zählung von magnetisch markierten Partikeln beschrieben. Dabei können diese magnetisch markierten Partikel auch über einen Magnetsensor geführt werden.

In dem Bericht "magnetoresistive Immunosensor for the detection of Escherichia coli O157:H5 including a microfluidic network"; M. Mujika et al.; Biosensors and Bioelectronics 24 (2009), S.1253-1258 ist ein tragbarer Sensor zur Bestimmung von Krankheitserregern auf Basis von einer magnetoresistiven Sensoranordnung beschrieben. Die Krankheitserreger werden mittels magnetischer Marker gekennzeichnet und anschließend mittels eines tragbaren Auswertegerätes detektiert.

Es ist Aufgabe der vorliegenden Erfindung, eine Vorrichtung zur magnetische Zelldetektion zu schaffen, die bei gleicher Präzision von Anreicherung und Messung eine Verkleinerung des Halbleitersubstrats, insbesondere eines Silizium-Chips, und dadurch auch eine Vereinfachung des Packaging der Messschaltung auf eine Platine ermöglicht.

Die Aufgabe der Erfindung ist durch eine Vorrichtung nach Patentanspruch 1 gelöst. Ein dazugehöriges Herstellungsverfahren ist in Patenanspruch 11 angegeben. Ein Verfahren zur magnetischen Zelldetektion ist im Patentanspruch 14 beschrieben. Vorteilhafte Ausgestaltungen der Erfindung sind Gegenstand der Unteransprüche.

Die Vorrichtung zur magnetischen Durchflusszytometrie umfasst einen magnetoresistiven Sensor, mittels dem magnetisch markierte Zellen detektiert werden können. Des Weiteren umfasst die Vorrichtung eine Durchflusskammer, insbesondere einen Mikrofluidikkanal, der ausgestaltet ist mit einer Zellsuspension durchströmt zu werden. Insbesondere weist der Mikrofluidikkanal dazu einen Einlass auf, durch den die Zellprobe in die Detektionsvorrichtung injiziert werden kann. Des Weiteren kann die Innenfläche des Mikrofluidikkanals, beispielsweise in ihrer Oberflächenbeschaffenheit, auf eine Zellprobe, insbesondere auf deren Viskosität, angepasst sein. Des Weiteren umfasst die Vorrichtung eine Anreicherungsstrecke, wobei die Anreicherungsstrecke mäanderförmig ausgestaltet ist. Die Anreicherungsstrecke verläuft dabei zweckdienlicherweise entlang des Mikrofluidikkanals. Würde die magnetisch markierte Zellprobe unmittelbar nach der Injektion auf oder über einen Magnetsensor geleitet, könnten natürlich nicht alle markierten Zellen erfasst werden, da zum Zeitpunkt der Injektion der Zellprobe in die Vorrichtung die magnetisch markierten Zellen in der Zellprobe noch ungeordnet und zufällig im gesamten Probenvolumen verteilt sind. Daher verläuft die Anreicherungsstrecke insbesondere in einem externen Magnetfeld, das z.B. durch einen Permanentmagneten erzeugt wird. In diesem Magnetfeld etwa erfahren die magnetisch markierten Zellen in der Zellsuspension eine magnetische Kraft, durch die sie z.B. in Richtung des Kanalbodens des Mikrofluidikkanals bewegt werden. Somit können die magnetisch markierten Zellen am Kanalboden angereichert werden und dann ausreichend nah über den magnetoresistiven Sensor geführt werden. Dadurch erst ist eine sichere, im Wesentlichen 100-prozentige Detektion jeder einzelnen magnetisch markierten Zelle gewährleistet. Je länger die Anreicherungsstrecke desto sicherer ist es, dass alle magnetisch markierten Zellen bis zum Zeitpunkt des Sensorübertritts am Kanalboden angereichert sind.

Der Vorteil der mäanderförmigen Anreicherungsstrecke ist der verringerte Platzbedarf und die dadurch ermöglichte Miniaturisierung der gesamten Messvorrichtung und mögliche Integrierung der gesamten Messvorrichtung auf einen Halbleiterchip.

Die Vorrichtung hat also den entscheidenden Vorteil durch die Verringerung des Platzbedarfs für die magnetophoretische Anreicherungsstrecke, die hohen Kosten eines Halbleitersubstrats, insbesondere eines teueren Silizium-Dies einzusparen. Auch wird durch ein geringes Aspektverhältnis des Dies eine einfache Prozessierung gewährleistet. Als "Die" wird beispielsweise der ungehäuste Halbleiter-Chip, ein integriertes elektronisches Bauelement, das Halbleiter- oder Sensor-Substrat bezeichnet.

Außerdem wird neben dem Halbleiterchip auch das gesamte Mikrofluidikvolumen reduziert, was zu einer großen Kostenersparnis und Vereinfachung der Sensorherstellung führt. Die längere Anreicherungsstrecke kann vorteilhaft dazu genutzt werden, die Durchflussgeschwindigkeit der Zellprobe zu erhöhen und somit entweder den Durchsatz zu erhöhen und/oder die benötigte Messzeit für eine Probe zu verringern.

Die Durchflusskammer, d.h. insbesondere der Mikrofluidikkanal weist z.B. einen Durchmesser von um die 1000 µm auf, was einem Vielfachen eines Zelldurchmessers entspricht. Prinzipiell können Kanaldurchmesser zwischen 30 µm und 30000 µm realisiert sein.

In einer vorteilhaften Ausgestaltung der Erfindung weist die Anreicherungsstrecke der Vorrichtung zur magnetischen Durchflusszytometrie magnetische Führungsstreifen auf. Diese sind insbesondere so angeordnet, dass sie die Zellen auf die Kanalbodenmitte hin lenken. Dies hat den Vorteil, dass die am Kanalboden angereicherten magnetisch markierten Zellen so auf z.B. eine zentrale magnetische Führungslinie am Kanalboden entlang ausgerichtet werden, dass bei Sensorübertritt eine Einzelzelldetektion gewährleistet wird. Des Weiteren erfüllen die magnetischen Führungslinien die Aufgabe, die magnetisch markierten Zellen so auszurichten, dass deren Streufeld ein möglichst großes Signal im Sensor hervorruft.

Besonders vorteilhaft ist es die magnetischen Führungsstreifen ferromagnetisch auszugestalten. Die magnetische Markierung der Zellen erfolgt insbesondere durch superparamagnetische Marker.

Die magnetischen Führungsstreifen der Anreicherungsstrecke dienen insbesondere dazu, die Zellen näher zur Kanalmitte zu führen. Besonders in den Krümmungsbereichen der mäanderförmigen Anreicherungsstrecke wird dies dadurch unterstützt, dass die magnetischen Führungsstreifen so angebracht sind, dass sie zur Kanalmitte hinweisen. Die Führung zur Kanalmitte hin wird deshalb vorgenommen, da am Ende der Anreicherungs- und Ausrichtungsstrecke der magnetoresistive Sensor oder z.B. ein Sensor-Array, mittig im Kanal angeordnet sind. Die gesamte Kanalbreite mit Einzelsensoren abzudecken, würde die Messelektronik verkomplizieren. Die magnetoresistiven Bauteile können unterhalb des Mikrofluidikkanals angeordnet sein, in der Kanalwand des Mikrofluidikkanals angeordnet sein oder auch innerhalb des Kanals angeordnet sein.

Die Vorrichtung weist insbesondere ein Substrat, beispielsweise ein Halbleitersubstrat auf, auf dem der magnetoresistive Sensor sowie der Mikrofluidikkanal und auch die Anreicherungsstrecke angeordnet sind. Dabei ist der magnetoresistive Sensor insbesondere als "integrierte Schaltung" auf dem Halbleitersubstrat integriert. Der Mikrofluidikkanal verläuft wiederum insbesondere entlang der Anreicherungsstrecke auf dem Substrat. Beispielsweise können auch die magnetischen Führungsstreifen der Anreicherungsstrecke auf dem Halbleiterchip integriert sein. Die integrierte Lösung der Vorrichtung auf einem Halbleiterchip hat den Vorteil der Kompaktheit und geringen Größe.

In einer vorteilhaften Ausgestaltung der Erfindung ist der Mikrofluidkanal so entlang der Anreicherungsstrecke angeordnet, dass eine durch den Mikrofluidikkanal strömende magnetisch markierte Zellprobe an den magnetischen Führungsstreifen ausgerichtet wird. Diese Anordnung hat eben den Vorteil, dass die Zellen neben der Anreicherung am Kanalboden eine Ausrichtung der Streufelder erfahren, die eine hochsensitive Einzelzelldetektion am Sensor ermöglicht.

Insbesondere weist die Vorrichtung dazu einen Magneten auf, der so mit der Vorrichtung angeordnet ist, dass eine durch den Mikrofluidikkanal strömende magnetisch markierte Zellprobe durch das Magnetfeld des Magneten am Kanalboden angereichert wird. Die magnetisch markierten Zellen sind dazu insbesondere superparamagnetisch markiert. D.h. insbesondere sind superparamagnetische Partikel an die Zellen angebunden. Durch das Magnetfeld des Magneten, insbesondere eines Permanentmagneten, erfahren die magnetisch markierten Zellen innerhalb der Zellsuspension eine Kraft, die sie in Richtung Kanalboden führt.

In einer weiteren vorteilhaften Ausgestaltung der Erfindung sind der Mikrofluidikkanal und der magnetoresistive Sensor so angeordnet, dass eine durch den Mikrofluidikkanal strömende magnetisch markierte Zellprobe über den Sensor geführt wird. Insbesondere ist also der Sensor innerhalb oder unterhalb des Mikrofluidikkanals angeordnet, so dass eine durch den Mikrofluidikkanal strömende Zellsuspension in jedem Fall oberflächennah über den Sensor geleitet wird. Zweckdienlicherweise ist der Sensor in der Richtung am Kanalboden oder an einer Kanalwand angeordnet in die das Magnetfeld des Anreicherungsmagneten die magnetisch markierten Zellen führt. Demnach sieht der Sensor insbesondere genau die Seite des Mikrofluidikkanals, an die die magnetisch markierten Zellen angereichert werden.

In einer vorteilhaften Ausgestaltung der Erfindung beträgt die Anreicherungsstrecke mindestens 12500 µm Streckenlänge, insbesondere mindestens 15000 µm. Beispielsweise kann auch eine Anreicherungsstrecke von 1 mm Länge ausreichen. Die erforderliche Mindestlänger der Anreicherungsstrecke kann auch 20000 µm oder bis zu 1 cm betragen. Die Einflussfaktoren auf die erforderliche Länge der Anreicherungs- und Ausrichtungsstrecke werden im Folgenden noch ausgeführt.

Es hat sich gezeigt, dass diese hohe Streckenlänge den Vorteil hat, dass auch hochkonzentrierte Zellproben am Ende der Anreicherungsstrecke so am Kanalboden angereichert und so durch die magnetischen Führungslinien der Anreicherungsstrecke ausgerichtet werden können, dass zum Zeitpunkt des Überstreichen des magnetoresistiven Sensors eine zuverlässige Einzelzellerkennung gewährleistet ist.

Für eine derart lange Anreicherungsstrecke ist das Substrat in seiner größten Ausdehnung insbesondere höchstens 18000 µm, zumindest höchstens 20000 µm. Beispielsweise beträgt das Substrat in seiner größten Ausdehnung nur maximal 10 mm. Dabei sind die meisten Halbleiter Dies rechteckig ausgesägte Waferstücke und die maximale Ausdehnung des Substrats ist demnach deren Diagonale. Dank der mäanderförmigen Anreicherungsstrecke benötigt diese nur einen geringen Platzbedarf auf dem Substrat. Dies ist besonders vorteilhaft, da der Einsatz von Halbleitersubstraten, insbesondere Silizium Dies mit hohen Kosten verbunden ist. Durch die mäanderförmige Anreicherungsstrecke ist demnach gewährleistet, dass bei geringer Halbleiterchipfläche eine ausreichend große Anreicherungsstrecke realisiert wird, mit der auch hochkonzentrierte Zellproben so angereichert und ausgerichtet werden können, dass die magnetisch markierten Zellen in diesen Zellproben durch den magnetoresistiven Sensor einzelnen detektiert werden können. Gleichzeitig verringert die Mäanderform der Anreicherungsstrecke das Aspektverhältnis des Substrats, was bedeutet, dass das Substrat kompakter wird und somit einfacher zu prozessieren ist.

Der magnetoresistive Sensor der Vorrichtung ist insbesondere ein GMR-Sensor (GMR = Giant Magneto Resistance bzw. Riesenmagnetowiderstand), Beispielsweise ist der magnetoresistive Sensor der Vorrichtung ein TMR-Sensor (TMR = Tunnel Magneto Resistance bzw. Tunnelmagnetowiderstand) oder der magnetoresistive Sensor der Vorrichtung ist ein AMR-Sensor (AMR = Anisotroper Magnetowiderstand).

In alternativen Ausführungsformen können auch optische Sensoren, wie Fluoreszenz- oder Streulicht-Sensoren zum Einsatz kommen oder diese mit magnetischen Sensoren kombiniert werden.

Bei dem Verfahren zur Herstellung einer oben beschriebenen Vorrichtung werden zunächst ein magnetoresistiver Sensor auf einem Substrat hergestellt, die magnetischen Führungsstreifen auf das Substrat aufgebracht und der Mikrofluidikkanal auf dem Substrat angebracht. In einer vorteilhaften Ausgestaltung des Herstellungsverfahrens wird der Sensor auf dem Halbleitersubstrat integriert. Dazu können bekannte Prozess-Verfahren der Mikrosystemtechnik eingesetzt werden.

In einer vorteilhaften Ausgestaltung des Herstellungsverfahrens werden die magnetischen Führungsstreifen der Anreicherungsstrecke direkt auf das Substrat abgeschieden, z.B. mittels thermischen Verdampfen oder Sputtern. Die magnetischen Führungsstreifen sind insbesondere aus einem ferromagnetischen Material, z.B. aus Nickel gefertigt. Auch ferromagnetische Legierungen können dazu eingesetzt werden.

Bei einem Messverfahren zur magnetischen Zelldetektion wird eine magnetisch markierte Zellprobe in eine oben beschriebene Vorrichtung mit mäanderförmiger Anreicherungsstrecke injiziert, innerhalb der Vorrichtung in einem Mikrofluidikkanal geführt, durch einen Magneten am Kanalboden so angereichert, dass die magnetisch markierten Zellen über den magnetoresistiven Sensor geführt und dort detektiert werden.

Die Anreicherung über ein externes Feld, z.B. das Feld eines Permanentmagneten, und die magnetophoretische Ausrichtung mittels der ferromagnetischen Führungsbahnen erfolgt bevorzugt in-situ während des Messvorgangs. Daher ist eine ausreichend lange Ausrichtungsstrecke für die magnetisch markierten Zellen erforderlich, um eine gewünschte Wiederfindungsrate von den markierten Zellen von im Wesentlichen 100 % zu gewährleisten. Einflussfaktoren auf die erforderliche Länge der Anreicherungs- und Ausrichtungsstrecke mit den ferromagnetischen Bahnen sind
1. die Geschwindigkeit mit der die Zellprobe durch den Mikrofluidikkanal gepumpt wird,
2. die Magnetfeldstärke des angelegten Anreicherungsmagnetfelds,
3. die Konzentration der superparamagnetisch markierten Zellen in der Suspension, sowie
4. die magnetischen Eigenschaften der eingesetzten Marker,
5. die Zusammensetzung und rheologischen Eigenschaften der Zellsuspension, d.h. z.B. deren Fließeigenschaft und
6. die Sorte der markierten Zellen und deren Isotopenanzahl auf der Zelloberfläche und damit die Anzahl der paramagnetischer Marker pro Zelle, die die Stärke des zu detektierenden Streufelds bestimmt.

Die Zellsuspension wird insbesondere mittels eines Druckgefälles durch den Mikrofluidikkanal gepumpt. Das Druckgefälle kann beispielsweise durch manuelle Bedienung einer Spritze oder eines Spritzensystems erzeugt werden. Dadurch ist gewährleistet, dass sich eine laminare Strömung der Zellprobe ohne Rezirkulation einstellt. Da die Zellen und das die Zellen umgebende komplexe Medium näherungsweise die gleiche Dichte aufweisen, tritt auch in den Krümmungsbereichen des mäanderförmigen Fluidikkanals nur eine geringe Fliehkraft auf und die markierten Zellen können auf ihrer Bahn verbleiben.

Die Vorrichtung und das Messverfahren damit sind von besonderem Vorteil für kleine Volumina hochkonzentrierter Proben (1000 Zellen pro µl), z.B. CD4+-Zellen. Im Blut eines gesunden Erwachsenen etwa machen die CD4+ T-Zellen ungefähr 25% - 60% der Lymphozyten aus. Eine Blutprobe würde demnach eine Konzentration von circa 300 -1600 Zellen/µl aufweisen. Eine Erhöhungen oder Verminderung der CD4+ T-Zellen kann bei vielen Erkrankungen vorkommen. Der Grad der Erhöhung oder Verminderung kann zwar nicht zum Nachweis einer Krankheit dienen jedoch ein Indikator dafür sein oder ergänzend eine bestehende Diagnose bestätigen. Beispiele bei denen eine Erhöhung der CD4+-Zellen auftritt sind Rheuma-Erkrankungen oder auch verschiedene Leukämien. Eine Verminderung der CD4+-Zellen kann ein Hinweis auf eine Immunschwäche, wie z.B. eine HIV-Erkrankung (AIDS) sein.

Bei der magnetischen Durchflusszytometrie ist also entscheidend, dass die magnetisch markierten Zellen sehr nah am magnetoresistiven Sensor vorbeitransportiert werden. Da die Zellprobe durch eine Durchflusskammer, z.B. einen Mikrofluidikkanal fließt, müssen die Zellen in dieser Durchflusskammer nahe an deren Innenfläche transportiert werden, wo der magnetoresistive Sensor angebracht ist. Insbesondere ist die Kanalwand in direktem Kontakt über dem Magnetsensor angebracht. In alternativen Ausführungsformen ist der magnetoresistive Sensor in die Kanalwand eingebettet. Als magnetische Markierung dienen bevorzugt superparamagnetische Labels. Als magnetoresistive Sensoren kommen GMR-, TMR- oder AMR-Sensoren in Frage. Die Nähe der magnetisch markierten Zelle zum Sensor ist deshalb so entscheidend, da das magnetische Streufeld der magnetischen Markierung im Nahfeldbereich mit dritter Potenz zum Abstand abfällt. Zusätzlich zur Anreicherung der magnetisch markierten Zellen an der Sensoroberfläche wirkt sich eine Ausrichtung der magnetisch markierten Zellen positiv auf deren Detektierbarkeit aus. Dabei werden die magnetisch markierten Zellen vorzugsweise so in Durchflussrichtung ausgerichtet, dass das Magnetfeld der magnetischen Markierung ein möglichst deutliches Signal im Sensor hervorruft. Bei der magnetischen Durchflusszytometrie ist eine möglichst genaue Differenzierung zwischen falschpositiven und positiven Signalen erforderlich. Dazu muss für positive Signale ein möglichst hoher Schwellwert für das Signal gesetzt werden können um sie von Rauschsignalen zu unterscheiden.

Im Gegensatz zur Methode, die magnetisch markierten Zellen vereinzelt über einen Sensor zu führen, indem sie in einem Mikrofluidikkanal durch dessen Durchmesser so eingeengt werden, dass nur einzelne Zellen diesen basieren können, weißt das Verfahren den Vorteil auf, eine im Wesentlichen 100%ige Einzelzelldetektion zu ermöglichen, direkt aus der unvorbereiteten komplexen Suspension heraus. Somit ist der große Nachteil der sozusagen mechanischen Vereinzelung der Zellen überwunden, dass diese zu Verstopfungen des Fluidiksystems führt. Auch könnten in einer derartigen Messvorrichtung keine magnetisch markierten Zellen von unterschiedlichem Durchmesser exakt einzeln bestimmt werden. Die Zellen haben beispielsweise Durchmesser im Bereich von ca. 3 bis 30 µm. Bevorzugt werden sie durch einen sehr viel breiteren Mikrofluidikkanal geführt, der im Durchmesser das 10- bis 1000fache beträgt. Der Sensor oder ein Sensor-Array von Einzelsensoren ist dabei quer zur Flussrichtung angeordnet und beträgt beispielsweise 30 µm Breite entsprechend dem Zelldurchmesser.

### Kurze Beschreibung der Zeichnung

Ausführungsformen der vorliegenden Erfindung werden in exemplarischer Weise mit Bezug auf die Figuren 1 bis 5 der angehängten Zeichnung beschrieben:
- Figur 1: zeigt eine mäanderförmige Anreicherungsstrecke,
- Figur 2: zeigt magnetische Führungslinien in der ersten Krümmung der Anreicherungsstrecke,
- Figur 3: zeigt eine alternative Magnetlinienanordnung in der ersten Krümmung der Anreicherungsstrecke,
- Figur 4: zeigt den Größenvergleich zwischen gerader und mäanderförmiger Anreicherungsstrecke,
- Figur 5: zeigt einen Querschnitt durch eine Messvorrichtung.

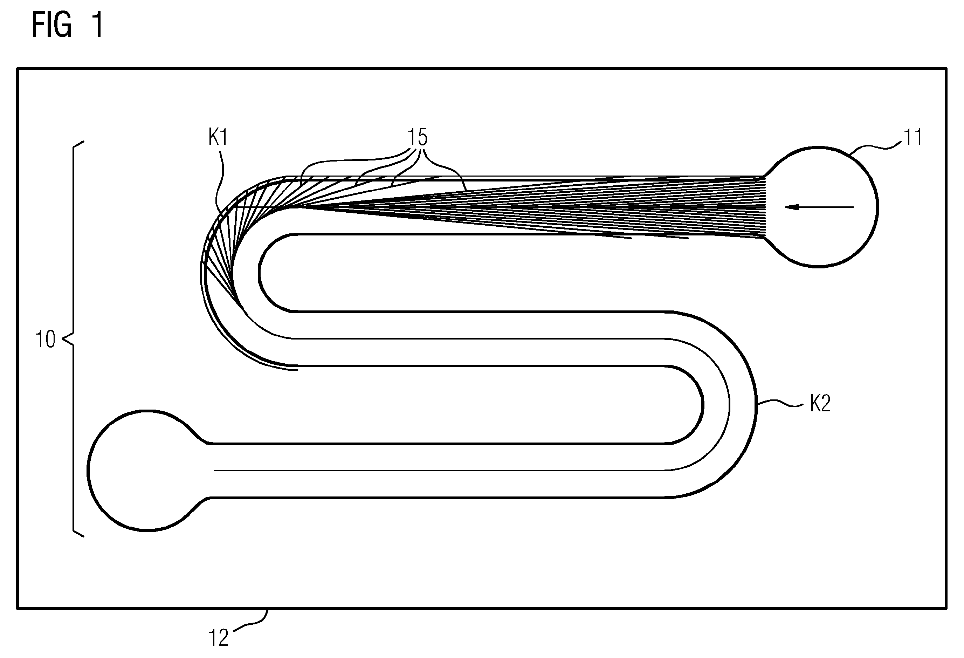

Die Figur 1 zeigt eine mäanderförmige Anreicherungsstrecke 10 gemäß einem Ausführungsbeispiel für die Erfindung. Die Anreicherungsstrecke 10 weist drei gerade Teilstrecken auf, die über zwei Krümmungen K1, K2 miteinander verbunden sind. Die Anreicherungsstrecke 10 ist zum einen zur Ausrichtung aber auch zur Anreicherung von magnetisch markierten Zellen 90 am Kanalboden ausgestaltet. D.h., in der gezeigten Draufsicht in Figur 1 ist ein Mikrofluidikkanal 50 so entlang der Anreicherungsstrecke 10 angebracht, dass eine Zellprobe, die durch diesen Mikrofluidikkanal 50 geleitet wird, die magnetischen Kräfte eines Permanentmagneten zur Anreicherung am Kanalboden sowie auch die magnetische Wechselwirkung mit den magnetischen Führungslinien 15 erfährt. Die in Figur 1 gezeigten magnetischen Führungslinien 15 laufen entlang der Anreicherungsstrecke 10 direkt auf dem Substrat 12, welches insbesondere die Oberfläche eines Halbleiterchips ist. Entlang der ersten geraden Teilstrecke laufen die magnetischen Führungslinien 15 in einem spitzen Winkel auf eine Mittellinie der Anreicherungsstrecke 10 zu und führen somit die magnetisch markierten Zellen 90 in die Kanalmitte. Entlang der ersten Krümmung K1 laufen die magnetischen Führungslinien 15 von dem Rand der Anreicherungsstrecke 10, d.h. auch vom Rand des Mikrofluidikkanals 50 zur Mitte der Anreicherungsstrecke 10 hin. In diesem Beispiel ist eine zentrale magnetische Führungslinie gezeigt, die stets entlang der Kanalmitte angeordnet ist. Des Weiteren zeigt die Figur 1 in der Draufsicht auf die Anreicherungsstrecke 10, einen Einlass 11 für eine Zellprobe in den Mikrofluidikkanal.

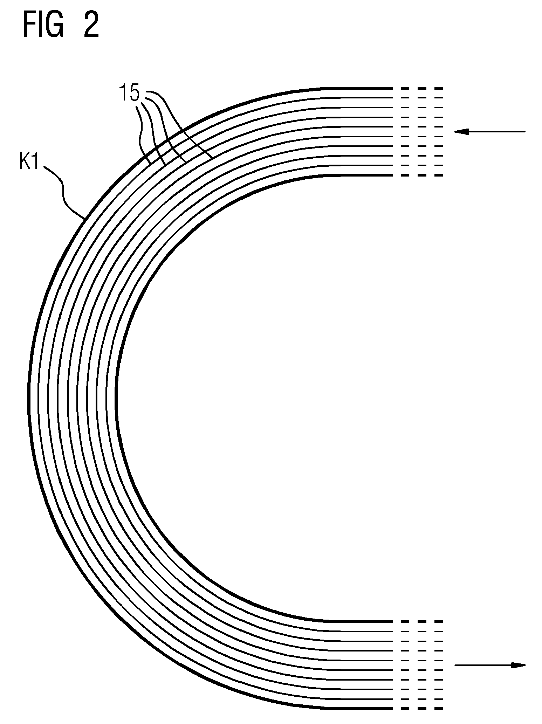

In der Figur 2 ist ein Ausschnitt der Anreicherungsstrecke 10 gezeigt mit der ersten Krümmung der Anreicherungsstrecke K1. In der Figur 2 ist eine alternative Ausführungsform der magnetischen Führungslinien 15 gezeigt. Diese können anstatt fächerförmig auf die Mittenlinie zuzulaufen auch halbkreisförmige Linien unterschiedlicher Radien sein, die jeweils in einem festen Abstand zu den Kanalwänden des Mikrofluidikkanals 50 eine Bahn beschreiben. In diesem Beispiel werden die magnetisch markierten Zellen 90 in der Zellprobe auf diesen Bahnen durch die Krümmung K1 geleitet. Die Pfeile deuten die Strömungsrichtung der Zellprobe durch die Krümmung K1 der Anreicherungsstrecke 10 an.

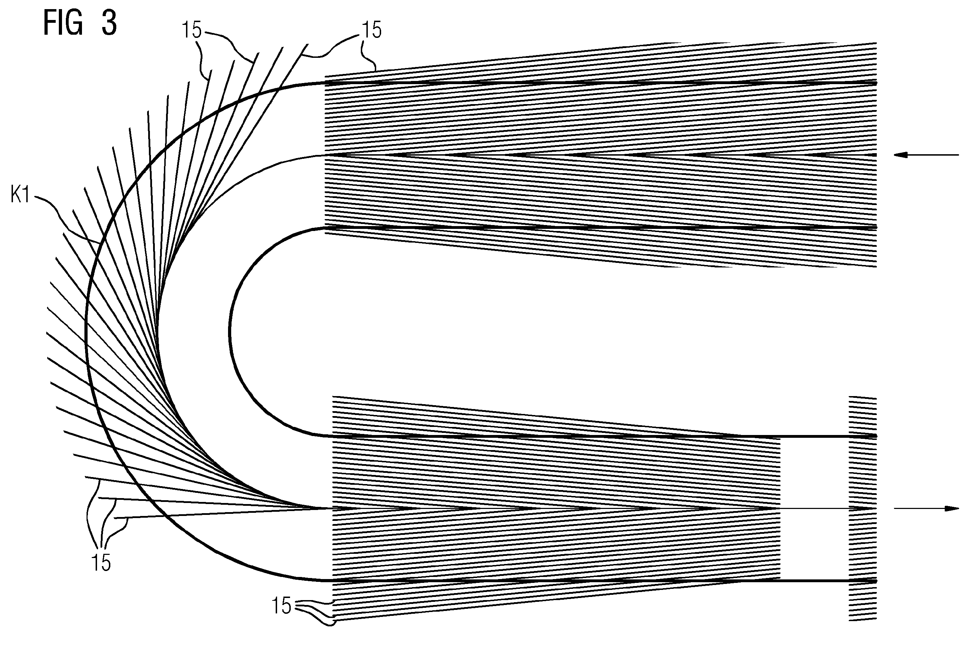

In Figur 3 ist ein größerer Ausschnitt der Anreichungsstrecke 10 gezeigt, der die erste Krümmung K1 sowie Teile der ersten und zweiten geraden Teilstrecke zeigt. Die magnetischen Führungslinien 15 zeigen in dieser Ausführungsform wieder ein fächerförmiges Bild. Sie führen von der Kanalwand zur Mittenlinie des Kanals 50 hin, sowohl in der Krümmung K1 als auch auf den geraden Teilstrecken. Auf den geraden Teilstrecken führen sie insbesondere in einem spitzen Winkel zur Mittenlinie des Kanals 50 hin. Die durch den Mikrofluidikkanal 50 bewegte Zellprobe 90 wird demnach zur Mitte des Kanals 50 hingeführt.

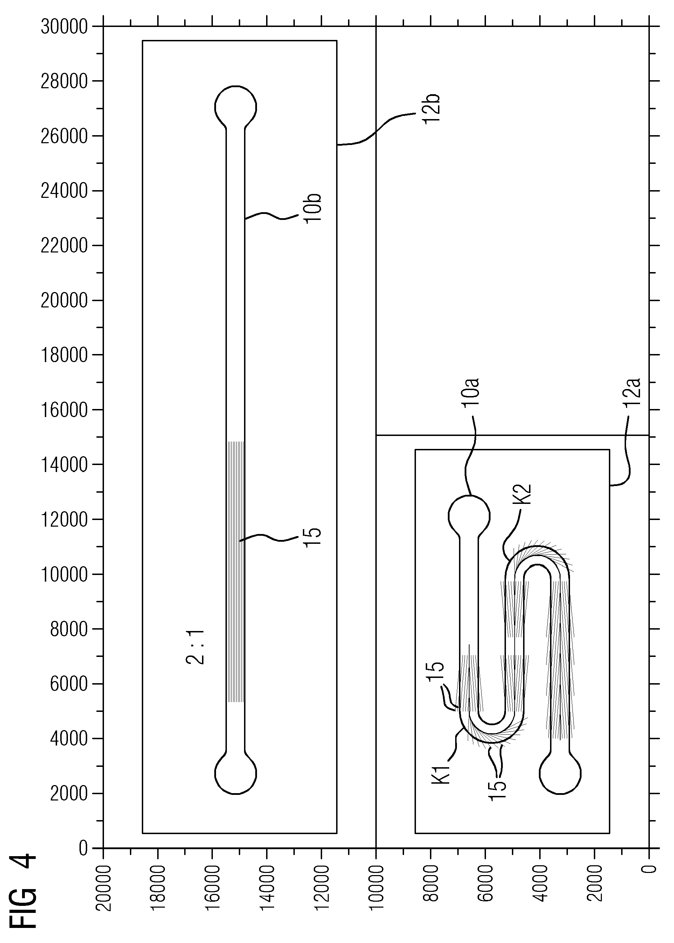

Figur 4 zeigt eine weitere Draufsicht auf die Anreichungsstrecke 10a im Vergleich mit einer linearen Anreicherungsstrecke 10b. Dazu sind Längenskalen in Mikrometer angegeben. Die Anreicherungsstrecke 10a weist die gleiche Gesamtlänge wie die lineare Anreicherungsstrecke 10b auf, jedoch benötigt diese einen nur halb so großen Halbleiterchip 12a als Substrat 12 auf dem die Anreicherungsstrecke 10a in Form der Magnetführungslinien 15 angeordnet ist.

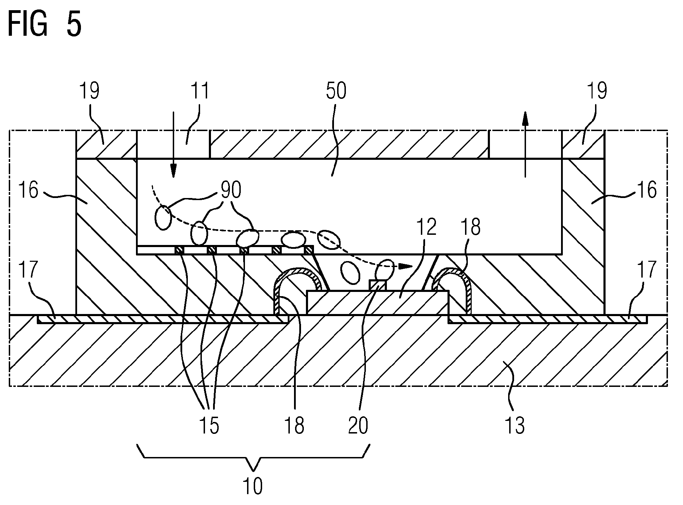

In der Figur 5 ist ein Querschnitt durch eine Ausführungsform der Messvorrichtung gezeigt, bei der die Anreicherungsstrecke 10 nicht direkt auf dem Halbleiterchip 12, sondern auf dem Packagingmaterial 16 ausgeformt ist. Im Querschnitt sind die magnetischen Führungslinien 15 gezeigt über die die magnetisch markierten Zellen 90 geführt werden. Insbesondere ist ober- oder unterhalb der Messvorrichtung ein Permanentmagnet angeordnet, über dessen Magnetfeld die Zellen 90 am Boden des Kanals 50 angereichert werden. Die Figur 5 zeigt des Weiteren einen Träger 13, auf dem Kontakte 17 abgeschieden sind. Auf dem Träger 13 wird der Halbleiterchip 12 aufgebracht und mittels Drahtbonden 18 an das Trägersubstrat 13 kontaktiert. Auf dem Halbleiterchip 12 befindet sich insbesondere ein magnetoresistiver Sensor 20 und ein kleiner weiterer Abschnitt einer Anreicherungsstrecke 600 mit Magnetführungslinien 15, die einen Offset 601zur Anreicherungsstrecke 10 auf dem Packagingmaterial 16 ausgleichen kann. Beispielsweise wird über ein Spritzgussverfahren mit dem Packagingmaterial 16 eine Durchflusskammer 50 ausgeformt. Durch Pfeile ist wieder die Strömungsrichtung der Zellprobe angedeutet bzw. der Einlass 11 in den Mikrofluidikkanal 50 gekennzeichnet.

## Patentansprüche

1. Vorrichtung zur magnetischen Durchflusszytometrie mit
- einem magnetoresistiven Sensor (20),
- einer Durchflusskammer (50), die ausgestaltet ist, mit einer Zellsuspension durchströmt zu werden,
und
- einer Anreicherungsstrecke (10) zur Ausrichtung und Anreicherung einer magnetisch markierten Zellprobe (90),
wobei
die Anreicherungsstrecke (10) mäanderförmig ausgestaltet ist.

2. Vorrichtung nach Anspruch 1, wobei die Anreicherungsstrecke (10) magnetische Führungsstreifen (15) aufweist.

3. Vorrichtung nach Anspruch 2, wobei die magnetischen Führungsstreifen (15) ferromagnetisch sind.

4. Vorrichtung nach einem der vorstehenden Ansprüche mit einem Substrat (12), insbesondere einem Halbleitersubstrat, wobei der magnetoresistive Sensor (20) sowie die Durchflusskammer (50), die insbesondere ein Mikrofluidikkanal ist, und die Anreicherungstrecke (10) auf dem Substrat (12) angeordnet sind.

5. Vorrichtung nach einem der vorstehenden Ansprüche 2 bis 4, wobei der Mikrofluidikkanal (50) so entlang der Anreicherungsstrecke (10) angeordnet ist, dass eine durch den Mikrofluidikkanal (50) strömende magnetisch markierte Zellprobe (90) an den magnetischen Führungsstreifen (15) ausgerichtet wird.

6. Vorrichtung nach einem der vorstehenden Ansprüche mit einem Magneten, wobei der Magnet so angeordnet ist, dass eine durch den Mikrofluidikkanal (50) strömende magnetisch markierte Zellprobe (90) durch das Magnetfeld des Magneten am Kanalboden angereichert wird.

7. Vorrichtung nach einem der vorstehenden Ansprüche, wobei der Mikrofluidikkanal (50) und der magnetoresistive Sensor (20) so angeordnet sind, dass eine durch den Mikrofluidikkanal (50) strömende magnetisch markierte Zellprobe (90) über den Sensor (20) geführt wird.

8. Vorrichtung nach einem der vorstehenden Ansprüche, wobei die Anreicherungsstrecke (10) mindestens 12500 µm beträgt.

9. Vorrichtung nach einem der vorstehenden Ansprüche, wobei das Substrat (12) in seiner größten Ausdehnung höchstens 18000 µm misst.

10. Vorrichtung nach einem der vorstehenden Ansprüche, wobei der magnetoresistive Sensor (20) einen GMR-, TMR- oder AMR-Sensor aufweist.

11. Verfahren zur Herstellung einer Vorrichtung nach einem der Ansprüche 1 bis 10 mit den folgenden Schritten:
Herstellung eines magnetoresistiven Sensors (20) auf einem Substrat (12),
Aufbringung von magnetischen Führungsstreifen (15) auf das Substrat (12),
Anbringung des Mikrofluidikkanals (50) auf dem Substrat (12).

12. Verfahren nach Anspruch 11, wobei der magnetoresistive Sensor (20) auf einem Halbleitersubstrat (12) integriert wird.

13. Verfahren nach Anspruch 11 oder 12, wobei die magnetischen Führungsstreifen (15) direkt auf das Substrat (12) abgeschieden werden, insbesondere mittels thermischem Verdampfen oder Sputtern.

14. Verfahren zur magnetischen Zelldetektion bei dem eine magnetisch markierte Zellprobe (90) in eine Vorrichtung nach einem der Ansprüche 1 bis 10 injiziert wird.

## Claims

1. Device for magnetic flow cytometry, comprising
- a magnetoresistive sensor (20),
- a flow chamber (50), which is configured for a cell suspension to flow therethrough,
and
- an enriching route (10) for aligning and enriching a magnetically marked cell sample (90),
wherein
the enriching route (10) has a meandering design.

2. Device according to Claim 1, wherein the enriching route (10) has magnetic guide strips (15).

3. Device according to Claim 2, wherein the magnetic guide strips (15) are ferromagnetic.

4. Device according to one of the preceding claims, comprising a substrate (12), more particularly a semiconductor substrate, wherein the magnetoresistive sensor (20) and the flow chamber (50), which more particularly is a microfluidic channel, and the enriching route (10) are arranged on the substrate (12).

5. Device according to one of preceding Claims 2 to 4, wherein the microfluidic channel (50) is arranged along the enriching route (10) in such a way that a magnetically marked cell sample (90) flowing through the microfluidic channel (50) is aligned at the magnetic guide strips (15).

6. Device according to one of the preceding claims, comprising a magnet, wherein the magnet is arranged in such a way that a magnetically marked cell sample (90) flowing through the microfluidic channel (50) is enriched by the magnetic field of the magnet on the channel floor

7. Device according to one of the preceding claims, wherein the microfluidic channel (50) and the magnetoresistive sensor (20) are arranged in such a way that a magnetically marked cell sample (90) flowing through the microfluidic channel (50) is guided over the sensor (20).

8. Device according to one of the preceding claims, wherein the enriching route (10) is at least 12 500 µm.

9. Device according to one of the preceding claims, wherein the substrate (12) measures at most 18 000 µm along its greatest extent.

10. Device according to one of the preceding claims, wherein the magnetoresistive sensor (20) has a GMR sensor, TMR sensor or AMR sensor.

11. Method for producing a device according to one of Claims 1 to 10, comprising the following steps:
producing a magnetoresistive sensor (20) on a substrate (12),
applying magnetic guide strips (15) on the substrate (12),
attaching the microfluidic channel (50) to the substrate (12).

12. Method according to Claim 11, wherein the magnetoresistive sensor (20) is integrated onto a semiconductor substrate (12).

13. Method according to Claim 11 or 12, wherein the magnetic guide strips (15) are deposited directly onto the substrate (12), in particular by thermal evaporation or sputtering.

14. Method for magnetic cell detection, in which a magnetically marked cell sample (90) is injected into a device according to one of Claims 1 to 10.

## Revendications

1. Dispositif destiné à la cytométrie en flux magnétique avec
- un capteur magnétorésistif (20),
- une chambre de flux (50), qui est conçue pour être traversée par une suspension cellulaire,
et
- un tronçon d'enrichissement (10) destiné à l'alignement et l'enrichissement d'une sonde cellulaire à marquage magnétique (90),
dans lequel le tronçon d'enrichissement (10) est conçu en forme de méandre.

2. Dispositif selon la revendication 1, dans lequel le tronçon d'enrichissement (10) présente des bandes de guidage magnétiques (15).

3. Dispositif selon la revendication 2, dans lequel les bandes de guidage magnétique (15) sont ferromagnétiques.

4. Dispositif selon l'une des revendications précédentes avec un substrat (12), en particulier un substrat à semiconducteur, dans lequel le capteur magnétorésistif (20) ainsi que la chambre de flux (50), qui est en particulier un canal microfluidique, et le tronçon d'enrichissement (10) sont disposés sur le substrat (12).

5. Dispositif selon l'une des revendications précédentes 2 à 4, dans lequel le canal microfluidique (50) est disposé le long du tronçon d'enrichissement (10) de sorte qu'une sonde cellulaire à marquage magnétique (90) traversant le canal microfluidique (50) est alignée sur les bandes de guidage magnétiques (15).

6. Dispositif selon l'une des revendications précédentes avec un aimant, dans lequel l'aimant est disposé de sorte qu'une sonde cellulaire à marquage magnétique (90) traversant le canal microfluidique (50) est alignée sur le fond de canal par le biais du champ magnétique de l'aimant.

7. Dispositif selon l'une des revendications précédentes, dans lequel le canal microfluidique (50) et le capteur magnétorésistif (20) sont disposés de sorte qu'une sonde cellulaire à marquage magnétique (90) traversant le canal microfluidique (50) est guidée via le capteur (20).

8. Dispositif selon l'une des revendications précédentes, dans lequel le tronçon d'enrichissement (10) est d'au moins 12500 µm.

9. Dispositif selon l'une des revendications précédentes, dans lequel le substrat (12) sur sa plus grande étendue mesure tout au plus 18000 µm.

10. Dispositif selon l'une des revendications précédentes, dans lequel le capteur magnétorésistif (20) présente un capteur GMR, TMR ou AMR.

11. Procédé destiné à la fabrication d'un dispositif selon l'une des revendications 1 à 10 avec les étapes suivantes :
fabrication d'un capteur magnétorésistif (20) sur un substrat (12),
application de bandes de guidage magnétiques (15) sur le substrat (12),
installation du canal microfluidique (50) sur le substrat (12).

12. Procédé selon la revendication 11, dans lequel le capteur magnétorésistif (20) est intégré sur un substrat à semiconducteur (12).

13. Procédé selon la revendication 11 ou 12, dans lequel les bandes de guidage magnétiques (15) sont séparées directement sur le substrat (12), en particulier au moyen de l'évaporation ou de la pulvérisation thermiques.

14. Procédé destiné à la détection cellulaire magnétique dans lequel une sonde cellulaire à marquage magnétique (90) est injectée dans un dispositif selon l'une des revendications 1 à 10.
